# EUROPEAN PATENT SPECIFICATION

(11) **EP 1 974 211 B1**
(45) Date of publication and mention of the grant of the patent: **20.09.2017**
(21) Application number: 07700431.5
(22) Date of filing: 18.01.2007
(51) Int. Cl.: G01N 33/50, G01N 33/53, G01N 33/543

(54) **MOLECULAR IDENTIFICATION THROUGH MEMBRANE-ENGINEERED CELLS**
MOLEKULARE IDENTIFIZIERUNG ÜBER MEMBRANKONSTRUIERTE ZELLEN
IDENTIFICATION MOLÉCULAIRE AU MOYEN DE CELLULES À MEMBRANE MODIFIÉE

(30) Priority: 20.01.2006 GR 20060100036
(43) Date of publication of application: 01.10.2008
(73) Proprietor: Kintzios, Spyridon, 11141 Athens (GR)
(72) Inventor: Kintzios, Spyridon, 11141 Athens (GR)
(74) Representative: Brown, Andrew Stephen
(86) International application number: PCT/GR2007/000004
(87) International publication number: WO 2007/083170

(56) References cited:
- EP-B1- 0 362 758
- WO-A-01/36965
- WO-A-99/30156
- WO-A-02/066683
- WO-A1-03/050524
- US-A- 5 789 152
- PAGE D L ET AL: "A cell-based immunobiosensor with engineered molecular recognition. III. Engineering molecular recognition" BIOSENSORS & BIOELECTRONICS ELSEVIER UK, vol. 12, no. 7, 1997, pages 559-566, XP002433006 ISSN: 0956-5663
- KINTZIOS S ET AL: "Study on the mechanism of Bioelectric Recognition Assay: evidence for immobilized cell membrane interactions with viral fragments" BIOSENSORS & BIOELECTRONICS, ELSEVIER SCIENCE PUBLISHERS, BARKING, GB, vol. 20, no. 4, 1 November 2004 (2004-11-01), pages 907-916, XP004633842 ISSN: 0956-5663
- PIZZICONI VINCENT B ET AL: "A cell-based immunobiosensor with engineered molecular recognition. Part I: Design feasibility" BIOSENSORS AND BIOELECTRONICS, vol. 12, no. 4, 1997, pages 287-299, XP002433007 ISSN: 0956-5663
- BURNS C J ET AL: "AMBRI biosensor - stabilizing artificial membranes and receptor attachment" PROC SPIE INT SOC OPT ENG; PROCEEDINGS OF SPIE - THE INTERNATIONAL SOCIETY FOR OPTICAL ENGINEERING 1999 SOCIETY OF PHOTO-OPTICAL INSTRUMENTATION ENGINEERS, BELLINGHAM, WA, USA, vol. 3858, 1999, pages 17-24, XP002433008
- REICHERT W M ET AL: "LANGMUIR-BLODGETT FILMS AND BLACK LIPID MEMBRANES IN BIOSPECIFIC SURFACE-SELECTIVE SENSORS" THIN SOLID FILMS, ELSEVIER-SEQUOIA S.A. LAUSANNE, CH, vol. 152, no. 1/2, 14 September 1987 (1987-09-14), pages 345-376, XP000500206 ISSN: 0040-6090
- MOSCHOPOULOU ET AL: "Application of ''membrane-engineering'' to bioelectric recognition cell sensors for the ultra-sensitive detection of superoxide radical: A novel biosensor principle" ANALYTICA CHIMICA ACTA, ELSEVIER, AMSTERDAM, NL, vol. 573-574, 28 July 2006 (2006-07-28), pages 90-96, XP005566931 ISSN: 0003-2670
- Ratna Chakrabarti ET AL: "Transfer of Monoclonal Antibodies into Mammalian Cells by Electroporation*", THE JOURNAL OF BIOLOGICAL CHEMISTRY, vol. 264, 15 September 1989 (1989-09-15), pages 15494-15500, XP055202621,
- K El Ouagari ET AL: "Electropermeabilization mediates a stable insertion of glycophorin A with Chinese hamster ovary cell membranes", European journal of biochemistry / FEBS, 1 February 1994 (1994-02-01), pages 1031-1039, XP055202785, GERMANY DOI: 10.1111/j.1432-1033.1994.tb18586.x Retrieved from the Internet: URL:http://www.ncbi.nlm.nih.gov/pubmed/811 2316
- NICOLAU C ET AL: "Electroinsertion of Proteins in the Plasma Membrane of Red Blood Cells", ANALYTICAL BIOCHEMISTRY, ACADEMIC PRESS INC, NEW YORK, vol. 214, no. 1, 1 October 1993 (1993-10-01), pages 1-10, XP024763781, ISSN: 0003-2697, DOI: 10.1006/ABIO.1993.1448 [retrieved on 1993-10-01]

## Description

The present invention relates to a method for the qualitative and/or quantitative determination of biological and chemical compounds, including microorganisms (such as viruses), on the basis of the specific and selective interaction of those compounds with one or more cells (cellular biosensors) the surface of which has been modified by the artificial insertion of molecules in such way that they react selectively with analytes under determination (molecular identification through membrane engineered cells).

In recent years there has been a rapid increase in the number of diagnostic applications based on biological sensors (biosensors). The reason for this is the tendency, in the case of routine analysis, to avoid bulky and heavy analytical instruments (e.g. liquid and gas chromatographers) with their concomitant high demand on trained personnel and laboratory infrastructure. In such cases (e.g. blood tests, monitoring of environmental pollution) the use of portable, easy-to-apply equipment is indicated as the method of choice. Determination of a particular molecule by using a biosensor operates on the principle of the (more or less) specific interaction of this molecule with a biological compound, such as a receptor or an enzyme and the subsequent indirect assay of this reaction. A particular group of biosensors are cell- or tissue biosensors, which are based on the use of live, intact cells (usually microorganisms) and have considerable applications for the detection of various biochemical agents due to their increased stability and lower cost of manufacture. However they often exhibit poor selectivity (Marty et al. 1998) which drastically reduces the scope of their application. Relatively recent methods have been developed for the improvement of selectivity. These are based on the genetic modification (transfection) of the cellular sensory material, so that cells express receptor molecules able to interact with particular substances under determination in an unknown sample. The following examples are characteristic:
(i) The CANARY system (Cellular Analysis and Notification of Antigen Risks and Yields) by Rider et al. (2003) that is based on genetically engineered human B cells and which has been applied for the detection of the pathogen *Yersinia pestis.*
(ii) The similar system by Whelan and Zare (2003), who developed a biosensor based on B cells expressing receptors for the constant region of immunoglobulin G (IgG).
(iii) The method of cell microarray transfection with plasmid DNA by Ziauddin and Sabatini (2001).

However, the use of transfected cells in cellular biosensors is neither a flawless process nor always applicable. For example, transient transfections result in the over-expression of individual genes, with the probability of producing a biased cellular phenotype, whereas not every cell line is amenable to transfection.

For this reason a novel biosensor principle is suggested, which comprises, as the main sensory material, cells (cellular biosensors) the surface of which has been modified by the artificial insertion of molecules in such way that they react selectively with analytes under determination (molecular identification through membrane engineered cells). The insertion of molecules can be achieved by standard methods, such as electroinsertion (Zeira et al., 1991). This approach is advantageous over using genetic engineering/transfection methods, since:
▪ Irrespective of the field of desired application, it is possible to find, isolate or manufacture (by appropriate methods of chemical synthesis) receptor-like molecules able to react specifically with different chemical or biological compounds, so that the desired selectivity is created. The receptor-like molecules may be proteins (such as enzymes, antigens or antibodies), nucleic acids, carbohydrates, lipids or belong to any other chemical group.
▪ There exists principally no limitation to the insertion of receptor-like molecules into the cell membranes. This can be achieved on cellular material originating either from natural sources or from in vitro culture or from artificial carriers (such as liposomes).
▪ It is possible to insert receptor-like molecules at a significantly higher density (per unit of cell surface) than in a solution or an immobilization system (e.g. a synthetic matrix or a membrane). In this way, an amplification of the reaction with a particular compound is achieved.
▪ Different types of receptor-like molecules can be inserted into the surface of the same cell, thus enabling combined reactions with sample components.

WO03/050524 describes the Bioelelectric Recognition Assay (BERA) in particular a method for indicating the presence of a material to be detected in a fluid comprising:
(a) bringing the fluid containing the material to be detected into contact with a detection region containing cellular material;
(b) monitoring an electrical property across the detection region; and
(c) detecting the presence of the material by sensing at least a predetermined change in the electrical property across the detection region. The biosensor is constructed using cellular material from cells which have been genetically engineered to possess receptors which bind specifically to a detectant of interest, e.g. cloned gene or genes with appropriate promotor/receptor may be inserted into a cell along with transmembrane sequences.

We have found that antibodies can be directly eletroinserted into the cell membrane of a Vero cell line in the correct orientation without any modification needed to the antibody. Accordingly we present as a feature of the invention a process for creating membrane engineered Vero cell line by electroinserting antibodies into the membrane of a Vero cell line.

The measurement of the interaction between an analyte and the appropriate receptor-like molecule is based on the following two parameters:
(i) The production of the reaction product (e.g. the product of an enzyme reaction)
(ii) The change of the membrane structure and/or function as a result of the interaction (e.g. the change of the cell membrane potential).

Consequently, the measurement of the interaction can be achieved by means of any appropriate method related to a physical chemical property of the biosensor, such as the measurement of the change of the electric potential (for example, by using the Biolelectric Recognition Assay(BERA)- Kintzios et al. 2001) or various optical properties (such as fluorescence, chemiluminescence or electrogenerated chemiluminescence).

The biosensor may comprise one or more cells, immobilized or not in a matrix or on a substrate of appropriate material and in such a way that the viability and functional integrity of the sensory material is preserved.

Therefore, the determination of a chemical or biological compound is possible provided that the pattern of a certain physical chemical property of the biosensor in response to various concentrations of this compound is known, relative to other compounds of similar structure or function.

The biosensor (in particular, the cell array in immobilized or non-immobilized state) is connected with any recording device appropriate for measuring the electric potential or any other selected physical chemical property of the sensor, including devices for the analog-to-digital conversion of signals and appropriate equipment and software for processing these signals, also as a part a continuous or real-time monitoring system.

Sample application can be done in any suitable way depending on the liquid or gas phase of the sample solution. The sample volume can be very small (< 5 µL). A solvent free of the compound under determination can be used as the reference solution (control). The effect of pH, conductivity, ionic strength, volume or other parameters of the sample solution on the biosensor response must always be taken under consideration. However, such effects could be minimized by using a suitable reference solution and assaying the response of a reference biosensor.

The procedure of biosensor construction and operation is briefly outlined in the following Example of an Application. It must be emphasized that biosensor construction takes place under sterile conditions in order to avoid the contamination of the cellular material.

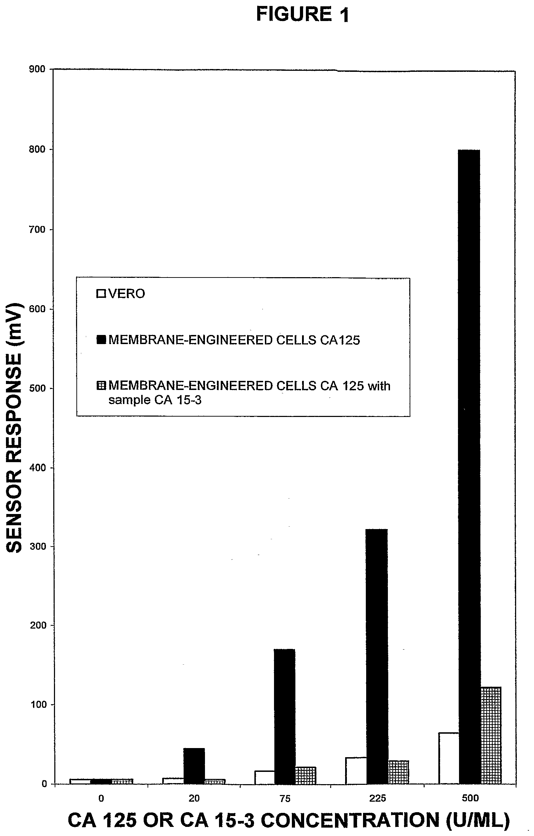

FIGURE 1 shows the results of the determination of the cancer biomarker CA 125 with a BERA biosensor based on membrane engineered cells (closed columns) compared to a BERA sensor based on conventional (non-engineered) cells (open columns). In addition, it shows the response of membrane-engineered cells to antigen CA 15-3, another cancer biomarker (shadowed columns).

In this application the method was used for the determination of the cancer biomarker antigen CA 125 in standard (calibration) solutions. The measurement of the interaction between the antigens and the antibodies, which have been incorporated into membrane-engineered cells, was based on the measurement of changes of the cell membrane potential according to the principles of the Bioelectric Recognition Assay (BERA). The biosensor was manufactured under sterile conditions by immobilizing African monkey kidney fibroblasts (Vero cell line). Membrane-engineered cells were constructed using the same cell line and inserting monoclonal antibodies against CA 125 by electroinsertion.

More analytically, Vero cells (at a density of 50,000/ml) were immobilized in 2% (w/v) calcium alginate beads, 2 mm wide, according to the protocol of Kintzios et al. (2003). Each bead was a cellular biosensor.

Membrane engineered cells were created by electroinserting monoclonal antibodies against CA 125 (1500 units/ml) into the membrane of Vero cells. Vero cells were incubated together with the antibody solution for 20 min on ice. Then, the cells-antibodies mixture was transferred to appropriate electroporator (Thermo EC100, Waltham, MA) cuvettes. Electroinsertion succeeded after applying an electric field (three pulses) at 400 V. After electroinsertion, cells were incubated at 37 °C for 1 hour, centrifuged at 100 g for 6 min and resuspended in 5 mM PBS (pH 7.4). This procedure was repeated enough times until no antibody was detectable in the supernatant.

Each cell-bearing bead (cell sensor) was connected to a working electrode made from pure silver, electrochemically coated with an AgCl layer. A cell-free bead was attached to the reference electrode. Electrodes were connected to the recording device, which comprised the PMD-1608FS A/D card (Measurement Computing, Middleboro, MA). The software responsible for the recording of the signal and processing of data was InstaCal (Measurement Computing).

For each assay, the sensor system, comprising of the beads attached to the working and the reference electrode, was immersed into the sample solution (1 ml). The response of each sensor was estimated by recording the change of the sensor potential after sample application and until the response was stabilized. A stable response of each biosensor was achieved 5-10 sec after sample addition. The average of the sensor potential of each assay was considered as the numerical value the responses of fifteen (15) biosensors.

The calibration standard included in the Chemiluminescent Microparticle Immunoassay of the commercial ARCHITECT kit (Abbott Laboratories, Chicago, IL, USA) was used as a sample (Batlle et al. 2005). The same method was used for comparing the results of the assay with membrane-engineered cells.

The results of the determination with the biosensor are presented in Figure 1. A definite selective response of the sensor to the sample, compared to the zero concentration control was observed. Sensors did not respond at all (null change of the steady-state potential) to control solutions or different concentrations of calibration solutions of antigen CA 15-3, another cancer biomarker. On the contrary, a linear response was observed within the range of determined concentrations of antigen CA 125, which was highly correlated with results derived with the commercial ARCHITECT kit (r²>0.97) (membrane-engineered cells are superior to the ARCHITECT kit in terms of low cost and speed of the assay procedure). Finally, Vero cells that had not been membrane engineered with the electroinsertion of the CA 125 antigen did not show any response to the addition of the samples.

The storage of the biosensors for a period of four months at room temperature did not affect measurements repeated at regular intervals.

The applications of the present invention include the detection and identification of molecules in biological and non-biological samples, such as the diagnosis of disease and infectious agents in medicine, veterinarian science and phytopathology, toxicology testing, analysis of metabolic products in living organisms, quality assurance through contaminant detection and monitoring of environmental pollution. These applications can be either commercial (in the sense of routine analyses) or serve pure research purposes.

Membrane-engineered cells can be used in cellular microarrays(comprising hundreds or thousands of cells) as well as for high throughput assays.

Some the advantages of the present invention, in comparison to other conventional or biosensory analysis methods, are the following:
▪ Ease of manufacturing and application, potentially for any compound under determination.
▪ Rapid assay.
▪ Low cost of each assay.
▪ Reusability of the biosensor, depending on the density of the immobilized cells and the degree of the reversible or not consumption of them during each assay.
▪ The sensor sensitivity can be increased by increasing the density of the cells (or artificial liposomes) and/or by increasing the concentration of artificially inserted receptor-like molecules, particularly when the cellular material is immobilized.

An important advantage of the invention is the fact that no prior knowledge is required of the exact interaction of the compound under determination with a particular receptor or enzyme or other cell system: the existence of molecules capable of a specific response to a compound is all that is required. In this way it is possible to construct biosensors for different applications in a short time.

Since the assay is rapid, the invention can be applied for the determination of compounds in real time measurements. For example, it can constitute part of a continuous monitoring system for environmental pollution, a chemical or biochemical reaction *in vivo* or the development of a disease in a host.

Although the present invention and its advantages have been described in detail, it should be understood that various changes, substitutions and alterations can be made herein without departing from the spirit and scope of the invention as defined by the appended claims.
Batlle M, Ribera JM, Oriol A, Pasor C, Mate JL, Fernandez-Avilez F, Flores A, Milla F, Feliu E, Leukemia & Lyphoma 2005, 46:1471-1476.
Kintzios S, Pistola E, Panagiotopoulos P, Bomsel M, Alexandropoulos N, Bem F, Varveri C, Ekonomou G, Biselis J, Levin R, Biosens. Bioelectron. 2001, 16: 325-336.
Kintzios S, Makri O, Panagiotopoulos Em, Scapeti M, Biotechnology Letters 2003, 25:405-408
Marty JL, Leca B, Noguer T, Analysis Magazine 1998, 26: 144-149.
Rider TH, Petrovick MS, Nargi FE, Harper JD, Schwoebel ED, Mathews RH, Blanchard DJ, Bortolin LT, Young AM, Chen J, Hollis MA, Science 2003, 301: 213-215.
Whelan RJ, Zare RN., Biosens. Bioelectron. 2003, 19: 331-336.
Zeira M., Tosi PF, Mouneimne Y, Lazarte J, Sneed L, Volsky DL, Nikolau C, 1991, Proc. Natl. Acad. Sci. USA 88: 4409-4415.
Ziauddin J., Sabatini DM, Nature 2001, 411; 107-109.

## Claims

1. A process for creating membrane engineered Vero cell line by electroinserting antibodies into the membrane of a Vero cell line.

2. A process as claimed in claim 1, wherein the Vero cells are incubated together with the antibody solutions for 20 minutes on ice.

3. A process as claimed in claim 1 or 2, wherein electroninsertion is succeeded after a Vero cell antibody mixture is transferred to an appropriate electroporator and applying three pulses of an electric field at 400v.

4. A process as claimed in claim 1 to 3 performed under sterile conditions.

5. A Vero cell line modified by the insertion of antibodies into the membrane and wherein the antibodies are inserted by electroinsertion.

6. A Vero cell line, as claimed in claim 5, which is immobilised in a matrix.

7. Use of a Vero cell line, as defined in claim 5 or 6, as a cellular biosensor.

8. Use as claimed in claim 7, wherein the cellular biosensor can measure the qualitative determination of the interaction of a chemical compound with an antibody by the measurement of a change of the electric potential of the cellular biosensor according to the principles of the Bioelectric Recognition Assay (BERA).

## Patentansprüche

1. Verfahren zum Erzeugen Membran-verarbeiteter Vero-Zelllinie durch elektrisches Einführen von Antikörper in die Membran einer Vero-Zellinie.

2. Verfahren nach Anspruch 1, wobei die Vero-Zellen für 20 Minuten auf Eis zusammen mit den Antikörperlösungen inkubiert werden.

3. Verfahren nach Anspruch 1 oder 2, wobei Electroinsertion nach Transfer einer Vero-Zellen-Antikörpermischung auf einen geeigneten Elektroporator und Anwendung von drei Pulsen eines elektrischen Feldes bei 400V gelungen ist.

4. Ein Verfahren nach einem der Ansprüche 1 bis 3, durchgeführt unter sterilen Bedingungen.

5. Eine Vero-Zelllinie modifiziert durch die Insertion von Antikörpern in die Membran, und wobei die Antikörper durch elektrisches Einführen eingefügt werden.

6. Eine Vero-Zellinie nach Anspruch 5, die in einer Matrix immobilisiert ist.

7. Verwendung der Vero-Zellinie, nach Anspruch 5 oder 6, als zelluläres Biosensor.

8. Verwendung nach Anspruch 7, wobei das zellulare Biosensor die qualitative Bestimmung die Interaktion einer chemischen Verbindung mit einem Antikörper durch die Messung einer Änderung des elektrischen Potentials des zellulären Biosensor nach den Prinzipien der Bioelekrtischen Anerkennungsprobe (BERA) messen kann.

## Revendications

1. Procédé pour la génération d'une lignée cellulaire Vero qui est modifiée par membrane ingénierie en électro-insérant les anticorps dans la membrane des cellules Vero.

2. Procédé selon la revendication 1, dans lequel les cellules Vero sont incubées avec des anticorps pendant 20 minutes sur la glace.

3. Procédé selon la revendication 1 ou 2, dans lequel l'électroinsertion est reussie après un mélange de cellules Vero et d'anticorps est transféré dans un électroporateur approprié et l'application de trois impulsions d'un champ électrique à 400V.

4. Procédé selon la revendication 1 à 3, est réalisé dans des conditions stériles.

5. Lignée cellulaire Vero modifiée par l'insertion d'anticorps dans la membrane et dans laquelle les anticorps sont insérés par de 'électroinsertion.

6. Lignée cellulaire Vero, tel que revendiquée dans revendication 5, qui est immobilisée dans une matrice.

7. Utilisation de la lignée cellulaire Vero, tel que défini dans la revendication 5 ou 6, en tant que biocapteur cellulaire.

8. Utilisation selon la revendication 7, dans lequel le biocapteur cellulaire peut mesurer la détermination qualitative de l'interaction d'un composé chimique avec un anticorps par la mesure d'une variation de potentiel électrique du biocapteur cellulaire selon les principes d'Essai de Reconnaissance Bio-électrique (BERA).
